# EUROPEAN PATENT SPECIFICATION

(11) **EP 0 878 189 B1**
(45) Date of publication and mention of the grant of the patent: **16.02.2005**
(21) Application number: 98108626.7
(22) Date of filing: 12.05.1998
(51) Int. Cl.: A61K 7/48, A61K 7/06, A61K 7/50, A61K 7/13, A61K 7/135, A61K 7/16, A61K 7/32, A61K 7/42, A61K 31/00, A61K 47/00, C08B 11/193

(54) **Hydrophobically modified polysaccharide in personal care products**
Hydrophob modifiziertes Polysaccharid in Körperpflegeprodukten
Dérivé hydrophobe de polysaccharide dans des produits de soin corporel

(30) Priority: 12.05.1997 US 855779
(43) Date of publication of application: 18.11.1998
(73) Proprietor: HERCULES INCORPORATED, Wilmington, Delaware 19894-0001 (US)
(72) Inventor: Modi, Jashawant J., Hockessin, Delaware 19707 (US)
(74) Representative: Hansen, Bernd, Dr. Dipl.-Chem.

(56) References cited:
- EP-A- 0 362 769
- WO-A-96/17916
- DE-A- 19 629 248
- DATABASE WPI Week 199428 Derwent Publications Ltd., London, GB; AN 1994-230614 XP002171983 "Aqua type gel base for mouth - comprising hydroxy ethyl cellulose, moisturising agent selected from glycerine, sorbitol or polyethylene glycol and water" & JP 06 166614 A (SHINETSU CHEM IND CO LTD), 14 June 1994 (1994-06-14)

## Description

This invention relates to the use of hydrophobically modified polysaccharides in personal care products. More specifically, this invention relates to the use of such polysaccharides in personal care products where the alkyl moiety of the hydrophobe has 1-7 carbon atoms.

Prior to the present invention, nonionic water soluble polysaccharides were used in personal care applications of shaving products, such as shaving creams and shaving gels, shampoos, shampoo conditioners, hair coloring systems, skin creams, lotions, facial cleansing products, under arm products, such as deodorants, antiperspirants, and mixtures thereof, lubricating gels, oral care products, such as toothpastes and mouth washes, denture adhesives, hair styling agents, such as hair gels and mousses, soaps, shower gels, body washes, make-up products, and sun screen products. Widely used commercially available polysaccharides include nonionic water soluble polysaccharide ethers such as methyl cellulose (MC), hydroxypropylmethylcellulose (HPMC), hydroxyethylcellulose (HEC), hydroxypropylcellulose (HPC), and ethylhydroxyethylcellulose (EHEC) and hydroxypropyl (HP) guar, hydroxyethyl guar, and HP starch and other nonionic starch and guar derivatives. Also, hydrophobically modified polysaccharides are used in personal care products. The use of these prior art polysaccharides in personal care products sometimes have processing difficulties such as compatibility with other ingredients, solubility with certain other ingredients, clarity (when needed) and stability under alkaline conditions of the personal care products. Also, hydrophobically modified polysaccharide are used in personal care products.

US Patent numbers 5,106,609, 5,104,646, and 5,100,658 are examples of patents that disclose the use of hydrophobically modified nonionic cellulose ethers in personal care products. These patents disclose the use high molecular weight (i.e., 300,000 to 700,000) and long chain alkyl carbon substitution in the hydrophobe (i.e., 8 to 24 carbons ) for use in hair and skin care cosmetics. Also, US Patent numbers 4,228,277 and 4,352,916 describe hydrophobically modified cellulose ether derivatives, modified with long chain alkyl group substitution in the hydrophobe. US Patent number 4,845,207 discloses a hydrophobically modified nonionic, water-soluble cellulose ether and US Patent 4,939,192 discloses the use of such ether in building **compositions.** EP-A-0 362 769 discloses mixed hydrophobe polymers having at least two hydrophobic long chain radicals and their use in aqueous protective coatings, such as latex paints.

Certain of the prior art nonionic cellulose ethers have poor compatibility with salts or poor solubility in certain solvents used in personal care applications such as polyhydric alcohols while others are not tolerant to alkaline conditions. Hence, a need still exists in the personal care industry to have nonionic cellulose ethers that have good compatibility with salts, good solubility in certain solvents, and tolerant to alkaline conditions while producing products that do not have color problems, when desired.
1. A personal care composition comprising a) from 0.1% to 99% by weight of a vehicle system which comprises a hydrophobically modified nonionic water soluble polysaccharide polymer which comprises a water soluble polysaccharide polymer backbone and a hydrophobic moiety selected from the group consisting of 3-alkoxy-2-hydroxypropyl group wherein the alkyl moiety is a straight or branch chain having 2-6 carbon atoms C₃-C₇ alkyl, aryl alkyl, alkyl aryl groups and mixtures thereof, wherein the ratio of the hydrophilic portion to the hydrophobic portion of the polymer is from 2:1 to 1000:1 and b) at least one other personal care ingredient.

It has been surprisingly found that hydrophobically modified polysaccharide having a short chain alkyl group in the hydrophobe moiety have various advantageous properties over prior art water soluble polysaccharide and their derivatives in personal care products. Any water soluble polysaccharide or derivatives can be used as the backbone to form the hydrophobically modified polysaccharide of this invention. Thus, e.g., hydroxyethylcellulose (HEC), hydroxypropyl cellulose (HPC), methylcellulose (MC), hydroxypropylmethylcellulose (HPMC), ethylhydroxyethylcellulose (EHEC), and methylhydroxyethylcellulose (MHEC) and, agar, dextran, locust bean gum, starch, guar and their nonionic derivatives can all be modified. The amount of nonionic substituent such as methyl, hydroxyethyl, or hydroxypropyl does not appear to be critical so long as there is a sufficient amount to assure that the ether is water soluble. The polysaccharides of this invention are nonionic having a sufficient degree of nonionic substitution to cause them to be water soluble and which are further substituted with a hydrocarbon radical having 1 to 7 carbon atoms in an amount up to the amount which renders said polysaccharide less than 1% by weight soluble in water.

The preferred polysaccharide backbone is hydroxyethylcellulose (HEC). The HEC which is modified to function in this invention is a commercially available material. Suitable commercially available materials are marketed by the Aqualon Division of Hercules Incorporated, Wilmington, Delaware U.S.A., under the trademark Natrosol®.

The short chain alkyl modifier can be attached to the polysaccharide backbone via an ether, ester, or urethane linkage. Preferred is the ether linkage as the reagents most commonly used to effect etherification are readily obtained, the reaction is similar to that commonly used for the initial etherification, and the reagents are usually more easily handled than the reagents used for modification via the other linkages. The resulting linkage is also usually more resistant to further reactions.

An example of the polysaccharides of the present invention is the 3-alkoxy-2-hydroxypropylhydroxyethylcellulose that is completely soluble in water at ambient temperature. Typically, the 3-alkoxy-2-hydroxypropylhydroxyethylcellulose used in this invention has a hydroxyethyl molar substitution (M.S.) the number of moles of hydroxyethyl substituent per cellulosic anhydroglucose unit in the cellulose molecule) of 1.5 to 3.5. The alkylglycidyl radical is generally contained in an amount of 0.05 to 50 wt. %, preferably 0.1 to 25 wt. %, based on the dry weight of the substituted polymer. Preferably the alkyl group of the 3-alkoxy-2-hydroxypropyl group is a straight chain alkyl group having 2 to 6 carbon atoms. Exemplary modifying radicals are methyl-, ethyl-, propyl-, butyl-, pentyl- and 2-ethylhexylglycidyl ether.

Generally, the preferred method for preparing the ethers of this invention comprises slurrying the nonionic polysaccharide in an inert organic diluent such as a lower aliphatic alcohol, ketone, or hydrocarbon and adding a solution of alkali metal hydroxide to the resulting slurry at a low temperature. When the ether is thoroughly wetted the reaction is continued, with agitation, until complete. Residual alkali is then neutralized and the product is recovered, washed with inert diluents, and dried. The etherification can also be affected with C₃ to C₇ halide or halohydride but these are sometimes less reactive, less efficient, and more corrosive so It is preferred to use the epoxide.

Substantially the same procedure is used to attach the hydrocarbon modifier via the ester or urethane linkage. Conventional slurry methods of reacting this type of modifier with polysaccharide, i.e., without the alkali, are ineffective. The alkali step is required in order to assure that the polysoccharide is swollen to the point that the modifier can react substantially uniformly on all polysaccharide molecules throughout. If reaction is not substantially uniform through the polysaccharide mass, the improved solubility and cloud point properties are not realized.

The hydrophoblcally modified polysaccharide of this invention show significantly improved salt tolerance in high salt systems compared to hydrophobically modified polysaccharide that are commercially marketed for personal care applications. In addition, these polysaccharides have improved solubility in solvent systems used in personal care applications compared to hydrophobically modified polysaccharide commercially marketed in the personal care industry. This salt tolerance of the polymer is determined by measuring the cloud point in a 15% sodium chloride solution. The cloud point is a temperature at which a clear solution starts to become cloudy and the polymer starts to precipitate out.

The hydrophobically modified hydroxyalkylcellulose of the present invention is an essential ingredient of the vehicle system of personal care products. In some products, it can be substantially the only ingredient needed for this vehicle system. Another ingredient that may be in the vehicle system is a surfactant that can be either soluble or insoluble in the composition. A compatible solvent may also be used in the vehicle system that can be either a single solvent or a blend of solvents.

Examples of the surfactants are anionic, nonionic, cationic, zwitterionic, or amphoteric type of surfactants. The surfactant can be insoluble (or soluble) in the present invention and (when used) is present in the composition of from 0.01 to 25% by weight of the composition.

Synthetic anionic surfactants include alkyl and alkyl ether sulfates. Specific examples of alkyl ether sulfates which can be used in the present invention are sodium coconut alkyl trimethylene glycol ether sulfate; sodium tallow alkyl trimethylene glycol ether sulfate; sodium tallow alkyl hexaoxyethylene sulfate; sodium tallow alkyl diethylene glycol ether sulfate; and sodium tallow alkyl sulfate.

Nonionic surfactants, can be broadly defined as compounds containing a hydrophobic moiety and a nonionic hydrophilic moiety. Examples of the hydrophobic moiety can be alkyl, alkyl aromatic, dialkyl siloxane, polyoxyalkylene, and fluoro-substituted alkyls. Examples of hydrophilic moieties are polyoxyalkylenes, phosphine oxides, sulfoxides, amine oxides, and amides.

Cationic surfactants useful in vehicle systems of the compositions of the present invention, contain amino or quaternary ammonium hydrophilic moieties which are positively charged when dissolved in the aqueous composition of the present invention.

Zwitterionic surfactants are exemplified by those which can be broadly described as derivative of aliphatic quaternary ammonium, phosphonium, and sulfonium compounds, in which the aliphatic radicals can be straight or branched chain, and wherein one of the aliphatic substituents contains from 8 to 18 carbon atoms and one contains as anionic water-solubilizing group, e.g., carboxy, sulfonate, sulfate, phosphate, or phosphonate.

Examples of amphoteric surfactants which can be used in the vehicle systems of the compositions of the present invention are those which are broadly described as derivatives of aliphatic secondary and tertiary amines in which the aliphatic radical can be straight or branched chain and wherein one of the aliphatic substituents contains from 8 to 18 carbon atoms and one contains an anionic water solubilizing group, e.g., carboxy, sulfonate, sulfate, phosphate, or phosphonate.

The water-soluble (or insoluble) surfactant is used with the polysaccharide of the present invention at from 0.01% to 25% of the composition.

According to the present invention, the solvent used in the vehicle system should be compatible with the other components in the present composition. Examples of the solvents used in the present invention are water, water-lower alkanols mixtures, and polyhydric alcohols having from 3 to 6 carbon atoms and from 2 to 6 hydroxyl groups. Preferred solvents are water, propylene glycol, water-glycerine, sorbitol-water, and water-ethanol. The solvent (when used) in the present invention is present in the composition at a level of from 0.1% to 99% by weight of the composition.

The active personal care component is optional because the vehicle system can be the active ingredient component. An example of this is the use of the vehicle system in a denture adhesive as either a cream or powder. However, when an active personal care ingredient is needed, it must provide some benefit to the user's body. Example of substances that may suitably be included in the personal care products according to the present invention are as follows:
1) Perfumes, which give rise to an olfactory response in the form of a fragrance and deodorant perfumes which in addition to providing a fragrance response can also reduce body malodor; 2) Skin coolants, such as menthol, menthyl acetate, menthyl pyrrolidone carboxylate N-ethyl-p-menthane-3-carboxamide and other derivatives of menthol, which give rise to a tactile response in the form of a cooling sensation on the skin; 3) Emollients, such as isosiopropylmyristate, silicone oils, mineral oils and vegetable oils which give rise to a tactile response in the form of an increase in skin lubricity; 4) Deodorants other than perfumes, whose function is to reduce the level of or eliminate micro flora at the skin surface, especially those responsible for the development of body malodor. Precursors of deodorants other than perfume can also be used; 5) Antiperspirant actives, whose function is to reduce or eliminate the appearance of perspiration at the skin surface; 6) Moisturizing agents, that keeps the skin moist by either adding moisture or preventing from evaporating from the skin; 7) Cleansing agents, that removes dirt and oil from the skin; 8) Sunscreen active ingredients, that protect the skin and hair from UV and other harmful light rays from the sun. In accordance with this invention a therapeutically effective amount will normally be from 0.01 to 10% by weight, preferable 0.1 to 5% by weight of the composition; 9) Hair treatment agents, that conditions the hair, cleans the hair, detangles hair, acts as styling agent, anti-dandruff agent, hair growth promoters, hair dyes and pigments, hair perfumes, hair relaxer, hair bleaching agent, hair moisturer, hair oil treatment agent, and antifrizzing agent; 10) Oral care agents, such as dentifrices and mouth washes, that cleans, whiten, deodorizes and protects the teeth and gum; 11) Denture adhesives that provide adhesion properties to dentures; 12) Shaving products, such as creams, gels and lotions and razor blade lubricating strips; 13) Tissue paper products, such as cleansing tissues; and 14) Beauty aids, such as foundation powders, lipsticks, and eye care.

The above list is only examples and is not a complete list of active ingredients that can be used in personal care compositions. Other ingredients that are use in these type of products are well know in the industry. In addition to the above ingredients conventionally used in products for personal care, the composition according to the present invention can optionally also include ingredients such as a colorant, preservative, antioxidant, vitamins, activity enhance, spermacidals, emulsifiers, viscosifying agents (such as salts, i.e., NaCl, NH₄Cl & KCI), and fats and oils.

The vehicle systems and personal care compositions of the present invention can be made using conventional formulation and mixing techniques. Methods of making various types of personal care compositions are described more specifically in the following examples.

In preferred embodiments of the present invention, the personal care composition is used in the following products: A hair or skin care composition, a shampoo, a conditioner, a shampoo-conditioner, a sun care product, a shower gel, a soap, a hair styling gel, a hair anti-dandruff composition, a hair growth promoter composition, a hair colorant composition, a hair bleaching agent, a hair antifrizzing agent, a hair relaxer composition, a dentifrice composition, a mouth wash composition, a denture adhesive composition, a shaving product composition, a lubricating gel composition, a spermicide gel composition, a beauty aid composition, an underarm solid stick composition, an underarm gel composition, an underarm liquid composition (optionally comprising an aerosol ingredient), a cleansing composition, a hair grooming and hair detangler composition, a raiser blade lubrication strip composition, and a cleansing tissue composition. The personal care composition of the present invention may also comprise an oil-in-water or water-in-oil emulsion.

### EXAMPLE 1

### Opaque Liquid Soap Formula

| **Ingredients** | **Weight %** |
|---|---|
| Water | 75.73 |
| Sodium C14-C16 olefin sulfonate, 40% active | 7.50 |
| Sodium lauroyl sarcosinate, 30% active | 6.66 |
| Cocamidopropyl betaine, 35% active | 6.66 |
| Glycol stearate | 1.00 |
| HMHEC1* | 0.80 |
| Propylene glycol | 0.50 |
| Glycerin | 0.50 |
| Tetrasodium EDTA | 0.30 |
| Stearalkonium chloride | 0.10 |
| Methylparaben | 0.25 |
| | $\overline{\text{100.00}}$ |

| | |
|---|---|
| *This compound is 3-butoxy-2-hydroxypropylhydroxyethylcellulose, that has aqueous viscosity at 25°C of a minimum of 2.5 Pa·s (2500 cps) at 1%, measured on a Brookfield LVTD Viscometer, and a cloud point of about 72°-78°C, that is treated with glyoxal. | |

### Procedure:

1. The HMHEC1* was dispersed in water. The pH was raised to about 8.0 - 8.5 to dissolve the polymer and mixed for 45 minutes. The methylparaben was added to the finished solution.
2. While slowly stirring the water-soluble polymer solution, the stearalkonium chloride, olefin sulfonate, and glycol stearate were added. The mixture was heated to 80°C until all of the glycol stearate was melted and the solution had turned opaque.
3. The remaining ingredients were added while cooling the solution slowly to room temperature.
4. The color and fragrance were added.

### COMPARATIVE EXAMPLE A

### Opaque Liquid Soap Formula

The same experiment was run as in Example 1 with the following changes. Supercol® U product was used in place of HMHEC1. The Supercol material was dispersed in water and mixed for 45 minutes. Next, methylparaben was added. The remaining procedure was the same.

### EXAMPLE 2

### Tollet Soap Formula

| **Ingredients** | **Weight %** |
|---|---|
| Water | 65.70 |
| Sodium C14-C16 olefin sulfonate | 20.00 |
| Sodium lauroyl sarcosinate | 10.00 |
| Cocamide MEA | 3.00 |
| HMHEC3* | 0.75 |
| Natrosol 250HR | 0.25 |
| Disodium EDTA | 0.20 |
| Methylparaben | 0.10 |
| | $\overline{\text{100.00}}$ |

| | |
|---|---|
| *This compound is 3-butoxy-2-hydroxypropylhydroxyethyl cellulose, that has an aqueous viscosity at 25°C of a minimum of 2 Pa·s (2000 cps) at 1%, measured on a Brookfield LVTD Viscometer, and a cloud point of about 62°-68°C with glyoxal treatment. | |

### Procedure:

1. The HMHEC3* and Natrosol 250HR product were dispersed in water. The pH was raised to about 8.0 - 8.5 to dissolve the polymer and mixed for 45 minutes. The methylparaben was added to the finished solution.
2. In a separate vessel, the surfactants were combined, heated to 80°C, and mixed until homogeneous.
3. The surfactant solution was added to the water-soluble polymer solution and mixed until well blended.
4. The disodium EDTA was added and cooled to room temperature.

### COMPARATIVE EXAMPLE B

### Toilet Soap Formula

The same experiment as in Example 2 was run with the exception that the Natrosol 250HR material was used in place of HMHEC3.

| **Source and Description of Products Used in Examples 1 and 2** | | |
|---|---|---|
| **Generic or CTFA Adopted Name** | **Trademark** | **Supplier** |
| Stearalkonium chloride | Ammonyx 4002 | Stepan Chemical Co. Northfield, Illinois |
| | | |
| Sodium C14-C16 olefin sulfonate | Bio-Terge AS-40 | Stepan Chemical Co. Northfield, Illinois |
| | | |
| Sodium lauroyl sarcosinate | Hamposyl L-30 | W. R. Grace & Co. Nashua, NH |
| | | |
| Cocamidopropyl betaine | Lexaine C | Inolex Chemical Co. Philadelphia, PA |
| | | |
| Cocamide MEA | Monamid CMA | Mona Industries Inc. Paterson, New Jersey |
| | | |
| Tetrasodium EDTA | Perma Kleer 100 | Stepan Chemical Co. Northfiel, Illinois |
| | | |
| | HMHEC 3 | Hercules Incorporated Wilmington, Delaware |
| | | |
| | HMHEC 1 | Hercules Incorporated Wilmington, Delaware |
| | | |
| | Natrosol 250HR | Hercules Incorporated Wilmington, Delaware |

### EXAMPLE 3

### Baby Hair Conditioner Formula

| **Ingredients** | **Weight %** |
|---|---|
| HMHEC3 | 1.0 |
| Water | 74.1 |
| Cetrimonium chloride (25%) | 12.2 |
| Lauramine oxide (30%) | 10.2 |
| Polyquaternium-17 (62%) | 1.5 |
| Propylene glycol | 1.0 |
| Perfume, preservative | *q*.*s*. to 100.0 |

### Procedure:

1. The HMHEC3 was dispersed in water with good agitation; and the pH was raised to about 8.0-8.5 to dissolve the polymer and the dispersion was mixed for 45 minutes or until the dispersion was fully dissolved.
2. The remaining ingredients were added in the order listed, mixing well between additions.

### COMPARATIVE EXAMPLE C

### Baby Hair Conditioner Formula

The same experiment as in Example 3 was run with the following exceptions. CMC 7HF was used in place of HMHEC3. The CMC was dispersed in the water and mixed for 45 minutes or until fully dissolved. The remaining ingredients were added in the order listed, mixing well between additions.

### EXAMPLE 4

### Pearlescent Cream Rinse Formulation

| **Ingredients** | | **Weight %** |
|---|---|---|
| Phase A. | HMHEC1 | 1.0 |
| | Natrosol 250HHR | 0.3 |
| | Water | 82.3 |
| Phase B. | Stearalkonium chloride (25%) | 10.1 |
| | Propylene glycol | 1.5 |
| | Glycol stearate | 1.5 |
| | Oleth-20 | 1.5 |
| | Polyquaternium-17 (62%) | 1.8 |
| | Perfume, preservative | *q*.*s*. to 100.0 |

### Procedure:

1. The HMHEC1 and Natrosol 250HHR product were dispersed in water with good agitation; the pH was raised to 8.0-8.5, and the dispersion was mixed until fully dissolved.
2. In a separate vessel, the stearalkonium chloride and propylene glycol were mixed together and heated to 80°C.
3. The other ingredients listed in Phase B except for the perfume and preservative were added one at a time in the order listed to the mixture of stearalkonium chloride and propylene glycol and mixed well between each addition.
4. The surfactant mixture of step 3 was added to the HMHEC1 solution, mixed well, and cooled to 35°C.
5. The perfume and preservative were then added to form the final formulation.

### COMPARATIVE EXAMPLE D

### Pearlescent Cream Rinse Formulation

The same experiment was run as in Example 4 except that CMHEC 420H was used in place of HMHEC1.

| **Raw Materials and Their Sources for Examples 3 and 4** | | |
|---|---|---|
| **CTFA Adopted Name** | **Trademark** | **Supplier** |
| Quaternium-48 | Adogen 470 | Sherex Chemical Co., Inc. Dublin, Ohio |
| | | |
| Oleth-20 | Emulphor ON-870 | Rhone-Poulenc Cranbury, New Jersey |
| | | |
| Hydrolyzed animal protein | Lexein X-250 | Inolex Chemical Company Philadelphia, Pennsylvania |
| | | |
| Polyquaternium-17 | Mirapol AD-1 | Rhone-Poulenc Cranbury, New Jersey |
| | | |
| Cocamidopropylamine oxide | Ammonyx CDO | Stepan Company Northfield, Illinois |
| | | |
| Lauramine oxide | Ammonyx LO | Stepan Company Northfield, Illinois |
| | | |
| Cetrimonium chloride | Varisoft E228 | Sherex Chemical Co., Inc. Dublin, Ohio |
| | | |
| Stearalkonium chloride | Varisoft SDC | Sherex Chemical Co., Inc. Dublin, Ohio |
| | | |
| Hydroxyethylcellulose hydroxyethylcellulose (as defined above) | Natrosol 250HHR | Hercules Incorporated Wilmington, Delaware |
| | | |
| Carboxymethylhydroxyethylcellulose | CMHEC420H | Hercules Incorporated Wilmington, Delaware |

### EXAMPLE 5

### Gentle Everyday Shampoo

| **Ingredients** | **Weight %** |
|---|---|
| Distilled water | *q*.*s*. to 100.00 |
| Sodium laureth sulfate, 28% | 19.60 |
| Cocamidopropyl betaine, 35% | 11.00 |
| Sodium lauroyl sarcosinate, 30% | 9.60 |
| PEG-150 distearate | 2.90 |
| HMHEC3 | 1.10 |
| Methylchloroisothiazolinone and Methylisothiazolinone, 1.5% | 0.08 |

### Procedure:

1. The HMHEC3 was dispersed by adding to the vortex of well-agitated, heated to 60-70°C, water in a vessel.
2. The surfactants, one at a time, were added to the vessel while mixing well between each addition.
3. The PEG-150 distearate was then added to the vessel, mixed until dissolved, and then the heat was turned off.
4. When the temperature cooled to 40°C or below, the fragrance and preservative were added to the formulation.

### COMPARATIVE EXAMPLE E

### Gentle Everyday Shampoo

The same experiment as in Example 5 was run except that the Natrosol Plus 430 product was used in place of HMHEC3.

| **Raw Materials and Their Sources** | | |
|---|---|---|
| **CTFA Adopted N** | **Trademark** | **Supplier** |
| Cocamidopropyl betaine | Lexaine C | Inolex Chemical Co. Philadelphia, Pennsylvania |
| | | |
| Methylchloroisothiazolinone And Methylisothiazolinone | Kathon CG | Rohm & Haas Co. Philadelphia, Pennsylvania |
| | | |
| Methyl paraben | Methyl Parasept | Kalama Chemicals. Inc. Gardfield, New Jersey |
| | | |
| PEG-150 distearate | Kessco PEG 6000 DS | Stepan Company Northfield, Illinois |
| | | |
| Sodium laureth sulfate, 28% | Steol 4N | Stepan Company Northfield, Illinois |
| | | |
| Sodium lauroyl sarcosinate | Hamposyl L-30 | W.R. Grace & Co. Lexington, Massachusetts |
| | | |
| Hydrophobically modified hydroxyethyl cellulose | Natrosol Plus® 420 | Hercules Incorporated Wilmington, Delaware |

### EXAMPLE 6

### Hand and Body Lotion

| **Part** | **Ingredients** | **Weight %** |
|---|---|---|
| A | HMHEC1 | 0.50 |
| | Distilled water | 78.25 |
| | Glycerin, USP | 2.00 |
| | | |
| B | Glycol stearate (Emerest® 2400) | 2.75 |
| | Stearic acid (Industrene® 5016K) | 2.50 |
| | Mineral oil (Drakeol® 7) | 2.00 |
| | Acetylated lanolin (Lipolan® 98) | 0.50 |
| | Cetyl alcohol (Crodacol® C95) | 0.25 |
| | | |
| C | Distilled water | 10.00 |
| | Triethanolamine | 0.50 |
| | | |
| D | Propylene glycol and diazolidinyl urea and Methylparaben & propylparaben (Germaben® II) | 0.75 |
| | | $\overline{\text{100.00}}$ |

### Procedure:

1. The *HMHEC4 was dispersed by adding to the vortex of well-agitated water in a vessel from Part A. The glycerin was then added with continued mixing and heated to 80°C for about 15 minutes.
2. In a separate vessel, the Part B ingredients were blended together, heated to 80°C, and mixed well.
3. The Part A and Part B components were slowly mixed together while agitating vigorously to produce an emulsion. This emulsion was maintained at a temperature of about 80°C with constant stirring.
4. Then, the Part C ingredients were added to the emulsion and the mixture was mixed continuously while cooling to 40°C.
5. The Part D (preservative) component was added to this new emulsion and was mixed well.
6. The formulation was then cooled and then poured into containers.

*HMHEC4 is 3-butoxy-2-hydroxypropylhydroxyethylcellulose, that has an aqueous viscosity at 25°C of a minimum of 500 mPa·s (500 cps) at 1.0%, measured on a Brookfield LVTD Viscometer, and a cloud point of about 62-68°C with glyoxal treatment.

### COMPARATIVE EXAMPLE F

### Hand and Body Lotion

The same experiment as in Example 6 was run except that Natrosol® 250MR was used in place of the HMHEC4.

| **Materials and Their Suppliers** | | |
|---|---|---|
| **CTFA Adopted Name** | **Trademark** | **Supplier** |
| Glycol stearate | Emerest 2400 | Henkel Corporation Hoboken, New Jersey |
| | | |
| Stearic acid | Industrene 5016K | Witco Corporation Newark, NJ |
| | | |
| Mineral Oil | Drakeol | Penreco Karns City, NJ |
| | | |
| Cetyl alcohol | Crodacol C-95 | Croda Inc. Parsippany, NJ |
| | | |
| Propylene glycol, diazolidinyl urea, Methylparaben and propylparaben | Germaben II | Sutton Laboratories Chatham, NJ |
| | | |
| Acetylated Lanolin | Lipolan 98 | Lipo Chemical Patterson, NJ |
| | | |
| Hydroxyethylcellulose | Natrosol 250MR | Hercules Incorporated Wilmington, DE |

### EXAMPLE 7

### Aerosol Shaving Cream

| **Ingredients** | **Amount, g** |
|---|---|
| Deionized water | 790.0 |
| Sodium hydroxide (24.6% solution) | 9.6 |
| Potassium hydroxide (34.2% solution) | 34.2 |
| Stearic acid, double pressed | 71.6 |
| Coconut acid | 10.0 |
| Propylene glycol | 27.0 |
| Lauramide DEA | 10.0 |
| Coconut oil | 2.5 |
| Tallow glycerides | 30.0 |
| Preservative (Germaben II) | 5.0 |
| HMHEC3 slurry | 10.0 |
| Total | $\overline{\text{1000.0}}$ |

### Procedure

To prepare the shaving cream concentrate, the sodium hydroxide and potassium hydroxide were added to the deionized water in a vessel at room temperature. The temperature of the vessel was then raised to 75°C and stirred for 5 minutes. The stearic acid and coconut acid were separately premelted and then each was added to the caustic/water mixture and then stirred for 30 minutes followed by cooling to 55°C. The HMHEC3 was slurried in the propylene glycol and added to the mixture. One at a time, the lauramide DEA (melted), coconut oil, tallow glycerides (melted), and preservative were added to the vessel and stirred for 15 minutes and allowed to cool.

This concentrate (225 g) was weighed into a standard 12-oz shaving cream can. The can was then sealed with a valve assembly using laboratory canning equipment and charged with 9.0 g of propellant.

### COMPARATIVE EXAMPLE G

### Aerosol Shave Cream

The same experiment as in Example 7 was repeated except that Natrosol® 250HR was used in place of HMHEC3.

| **List of Ingredients and Their Suppliers** | | |
|---|---|---|
| **CTFA Adopted Name** | **Trademark** | **Supplier** |
| Stearic acid | Industrene 5016 | Witco Corporation Memphis, Tennessee |
| | | |
| Coconut acid | Industrene 328 | Witco Corporation Memphis, Tennessee |
| | | |
| Lauramide DEA | Standamid LD | Henkel Corporation Ambler, Pennsylvania |
| | | |
| Coconut oil | Coconut oil | Sigma Chemical Co. St. Louis, Missouri |
| | | |
| Tallow glycerides | Peacock Acidless Tallow | Geo. Pfau's Sons Co. Jeffersonville, Indiana |
| | | |
| Sorbitol | Sorbo (70% active) | ICI Americas, Inc. Wilmington, Delaware |
| | | |
| Propylene glycol (and) Diazolidinyl urea (and) Methylparaben (and) Propylparaben | Germaben II | Sutton Laboratories Chatham, New Jersey |
| | | |
| 88/12 Isobutane/propane | A-46 Propellant | Aeropres Corporation Shreveport, Louisiana |
| | | |
| Propylene glycol | Propylene Glycol | Eastman Chemical Co. Rochester, New York |
| | | |
| Hydroxyethylcellulose | Natrosol 250 HR | Hercules Incorporated Wilmington, Delaware |

### EXAMPLE 8

### Standard Cream Toothpaste with HMHEC2

| | **Ingredient** | **wt%** |
|---|---|---|
| I. | HMHEC2* | 0.75¹ |
| | Glycerin 100% | 13.00 |
| | Sorbitol (70% solids) | 16.86 |
| | Distilled water | 15.61² |
| | | |
| II. | Dicalcium phosphate, anhydrous | 45.00 |
| | | |
| III. | Tetra sodium pyrophosphate | 0.42 |
| | Sodium saccharin | 0.20 |
| | Sodium monofluorophosphate | 0.76 |
| | Sodium benzoate | 0.50 |
| | Distilled water | 6.25 |
| | | |
| IV. | Flavor | 0.55 |
| | Sodium lauryl sulfate | 0.10 |
| | | $\overline{\text{100.00}}$ |

| | | |
|---|---|---|
| ¹ Correct polymer weight for moisture content. | | |
| ² For water: Adjust the amount of water for moisture in the polymer. | | |
| *HMHEC2 is 3-butoxy-2-hydroxypropylhydroxyethylcellulose that has an aqueous viscosity at 25°C of a minimum of 2 Pa·s (2000 cps at 1 %, measured on a Brookfield LVTD Viscometer and has a cloud point of about 62°-68°C without glyoxal treatment. | | |

### Procedure:

1. The salts of Part III were added to the water in a vessel while stirring and heated to about 60°C to dissolve. The vessel was covered during heating to prevent moisture loss.
2. Part I. The glycerine was weighed into a beaker and the polymer was dispersed in the glycerine while stirring for about 5 minutes or until adequately dispersed. Sorbitol was added and the mixture was continuously stirred for another 10 minutes. Water was added and stirred for an additional 15 to 30 minutes making sure that the polymer was completely hydrated (no gels). A warm salt solution was added while stirring continuously for an additional 15 minutes or until homogenous (no lumps or gels). This mixture was then transferred to a toothpaste mixer (Ross double planetary mixer).
3. Part II. The DCP and water were added to a mixer and mixed for 10 min. at a low speed to completely wet the DCP. The mixer was then opened and the DCP paste mixture was scraped from the beaters and bowl sides. The mixer was then closed and a vacuum was applied. The mixer was run on high speed under vacuum for 20 minutes or until the DCP paste mixture had a smooth consistency.
4. Part IV. The SLS was added to the mixer and mixed for 5 minutes at low speed without vacuum. The flavor was added to the mixer and mixed for 2 min. at low speed. The mixer was then opened and the beaters and bowl sides were scraped down. The mixer was closed and a vacuum was applied and mixed at medium speed for 15 minutes, observing for foaming.
5. The mixer was then shut off and the vacuum was broken and the formulation was packed out in a container as a paste.

### COMPARATIVE EXAMPLE H

### Standard Cream Toothpaste with HMHEC2

The same experiment as in Example 8 was repeated except that Natrosol 250 HR from Hercules Incorporated was used in place of HMHEC2.

### EXAMPLE 9

### Standard Cream Toothpaste with HMHEC2

| | **Ingredient** | **wt%** |
|---|---|---|
| I. | HMHEC2 CMC | 0.75¹ |
| | 7MF (Hercules Incorporated) | 0.25¹ |
| | Glycerin 100% | 13.00 |
| | Sorbitol (70% solids) | 16.86 |
| | Distilled water | 14.46² |
| | | |
| II. | Dicalcium phosphate, anhydrous | 45.00 |
| | | |
| III. | Tetra sodium pyrophosphate | 0.42 |
| | Sodium saccharin | 0.20 |
| | Sodium monofluorophosphate | 0.76 |
| | Sodium benzoate | 0.50 |
| | Distilled water | 6.25 |
| | | |
| IV. | Flavor | 0.55 |
| | Sodium lauryl sulfate | 1.00 |
| | | $\overline{\text{100.00}}$ |

| | | |
|---|---|---|
| ¹ Correct polymer weight for moisture content. | | |
| ² For water: Adjust the amount of water for moisture in the polymer. | | |

1. Begin with Part III. The salts were added to the water while stirring and heated to about 60°C to dissolve. The salt and water mixture was covered during heating to prevent moisture loss.
2. Part I. Glycerine was weighed into a beaker and the polymer was dispersed in the glycerine while stirring. This mixture was stirred for 5 minutes or until adequately dispersed. Sorbitol was added the this dispersion and was continuously stirred for another 10 minutes. Water was added and stirred for 15 to 30 minutes making sure that the polymer was completely hydrated (no gels). Then warm salt solution was added while stirring and was continuously stirred for 15 minutes or until homogenous (no lumps or gels). This mixture was then transferred to a toothpaste mixer (Ross double planetary mixer).
3. Part II. DCP was added to the mixer and was mixed for 10 min. at a low speed to completely wet the DCP. The mixer was then opened and beaters and bowl sides were scraped down. The mixer was closed and a vacuum was applied. The mixer was then run on high speed under vacuum for 20 minutes or until the mixture was smooth.
4. Part IV. SLS was added and mixed for 5 minutes at low speed without vacuum. Flavor was add and mixed for 2 min. at low speed and the mixer was opened and beaters and bowl sides were scraped down. The mixer was closed and a vacuum was applied. The mixer was run on medium speed for 15 minutes, observe for foaming.
5. The mixer turned off and the vacuum was broken. The blend from the mixer was packed out as a paste.

### EXAMPLE 10

### Cream Toothpaste with HMHEC1

| | **Ingredient** | **%** | **wt(g)** |
|---|---|---|---|
| I. | HMHEC1 | 0.75 | 15.00 |
| | Glycerine 100% | 13.00 | 260.00 |
| | Sorbitol (70%) | 16.86 | 337.20 |
| | Distilled water | 16.96² | 339.2 |
| | | | |
| II. | Dicalcium phosphate, dihydrate | 45.00 | 900.00 |
| | | | |
| III. | Tetra sodium pyrophosphate | 0.42 | 8.40 |
| | Sodium monofluorophosphate | 0.76 | 15.20 |
| | Sodium saccharin | 0.20 | 4.00 |
| | Sodium benzoate | 0.50 | 10.00 |
| | | | |
| IV. | Flavor | 0.55 | 11.00 |
| | | | |
| V. | Sodium lauryl sulfate | 1.00 | 20.00 |
| | Distilled water | 4.00 | 80.00 |
| | | $\overline{\text{100.00}}$ | $\overline{\text{2000.00}}$ |

| | | | |
|---|---|---|---|
| ¹ correct polymer weight for moisture content. ²Adjust the water level for polymer moisture correction. | | | |

1. Part I. The glycerine was weighed into a beaker. The polymer was dispersed in the glycerine in a Jiffy mixer while stirring. This glycerine and polymer mixture was stirred for 5 minutes or until adequately dispersed. Sodium saccharin and sodium benzoate were added to the dispersion and mixed for an additional 5 min. Sorbitol was then added and mixed for 5 min. Water was then added and stirred for 30 min. After stirring for the 30 min., total weight of beaker was recorded and stirred again. The solution was heated to 80°C and mixed for 15 min. at 80°C. The beaker was reweighed and the weight was adjusted for any weight loss due to evaporation. The contents of the beaker then was transferred to a Ross planetary vacuum mixer.
2. During the polymer hydration period, Part V was begun. SLS was added to the water while stirring and dissolved by warming to -50°C in a water bath, if lumping occurred, the process was restarted.
3. Part II. DCP was added to a mixer and was mixed for 10 min. on a low speed to completely wet the DCP. The mixer was stopped and the beaters and bowl sides were scraped down. The mixer was then closed and a vacuum was applied. The mixer was run on high speed under vacuum for 20 minutes or until smooth paste.
4. TSPP was added to the mixer and was mixed for 5 min. Then, SMFP as added and mixed for 5 min. The saccharin was added and mix for 5 mins. The sodium benzoate was added and mix 5 mins. on low speed followed by 10 min. on medium speed or until smooth.
5. Part IV. The SLS was added and mixed for 5 minutes on low speed without vacuum. The flavor was added and mixed for 2 min at low speed. The mixer was opened and the beaters and bowl sides were scraped down. The mixer was closed and a vacuum was applied and mixed at medium speed for 15 minutes, observe for foaming.
6. The mixer speed was reduced and shut off after awhile and the vacuum was broken. The mixer content was then packed out as a paste.

### EXAMPLE 11

### Cream Toothpaste with HMHEC1

| | **Ingredient** | **%** | **wt(g)** |
|---|---|---|---|
| I. | HMHEC1 | 0.75 | 15.00 |
| | Genuvsco TPH1 (Hercules Incorporated) | 0.25 | 5.00 |
| | Glycerine 100% | 13.00 | 260.00 |
| | Sorbitol (70%) | 16.86 | 337.20 |
| | Distilled water | 16.71² | 334.2 |
| II. | Dicalcium phosphate, dihydrate | 45.00 | 900.00 |
| III. | Tetra sodium pyrophosphate | 0.42 | 8.40 |
| | Sodium monofluorophosphate | 0.76 | 15.20 |
| | Sodium saccharin | 0.20 | 4.00 |
| | Sodium benzoate | 0.50 | 10.00 |
| IV. | Flavor | 0.55 | 11.00 |
| V. | Sodium lauryl sulfate | 1.00 | 20.00 |
| | Distilled water | 4.00 | 80.00 |
| | | $\overline{\text{100.00}}$ | $\overline{\text{2000.00}}$ |

| | | | |
|---|---|---|---|
| ¹ Correct polymer weight for moisture content. ²Adjust the water level for polymer moisture correction. | | | |

1. Part I. glycerine was weighed into a beaker. Polymer was dispersed in glycerine in a Jiffy mixer while stirring and was stirred for 5 minutes or until adequately dispersed. Sodium saccharin and sodium benzoate were added to the mixer and mix for 5 min. Sorbitol was then added to the mixer and mixed for 5 min. Water was then added and stir for 30 min. After stirring for 30 min., the total weight of beaker was recorded. The solution was then heated to 80°C., mixed for 15 min. at 80°C., and reweighed. The weight was adjusted for any weight loss due to evaporation. The mixture was transferred to a Ross planetary vacuum mixer.
2. During the polymer hydration period, Part V was begun. SLS was added to the water while stirring. The SLS was dissolved by warming to ∼50°C in a water bath. If lumping occurred, the process was restarted.
3. Part II. DCP was added to the mixer and mixed for 10 min. at a low speed to completely wet the DCP. The mixer was stopped and beaters and bowl sides were scraped down. The mixer was closed, a vacuum was applied, and was mixed on high speed under vacuum for 20 minutes or until the paste became smooth.
4. TSPP was added to the mixer and mixed for 5 min. Next the SMFP was added and mixed for 5 min. The saccharin was then added and mixed for 5 mins. Next, the sodium benzoate was added and mixed for 5 mins. on low speed followed by 10 min. on medium speed or until smooth.
5) Part IV. The SLS was added and mixed for 5 minutes on low speed without vacuum. Then, the flavor was added and mixed for 2 min at low speed. The mixer was then opened and the beaters and bowl sides were scraped down. The mixer was then closed and a vacuum was applied. The mixer was run at medium speed for 15 minutes, observe for foaming.
6. Mixer's speed was reduced gradually and finally shut off and the vacuum was broken. The content of the mixer was packed out as a paste.

### EXAMPLE 12

### Lubricating Jelly (or liquid)

### FORMULATION 1

2.2% Nonoxynol 9
3.0% HMHEC2
94.9% Propylene Glycol
0.1% Methyl parasept

### FORMULATION 2

2.2% Nonoxynol 9
1.5% HMHEC2
1.5% Natrosol 250HHX (Hercules Incorporated)
24.9 Water
70.9% Propylene Glycol
0.1% Methyl parasept

### FORMULATION 3

4.0% HMHEC2
95.9% Propylene Glycol
0.1% Methyl parasept

### FORMULATION 4

2.0% HMHEC2
1.5% Klucel HF (Hercules Incorporated)
94.9% Propylone Glycol
0.1% Methyl parasept

### FORMULATION 5

0.5% HMHEC2
0.5% Klucel HF
10.0% mineral oil
34.9% Propylene Glycol
54.0% water
0.1% Methyl parasept

The polymer was dispersed into vortex of vigorously agitated propylene glycol and/or mineral oil and mixed for ten minutes. Water was added. Next the temperature was raised to 90°C and mixed for one hour and then was gradually cooled to about 25°C, while mixing slowly. Nonoxynol and preservative (as required) were added while mixing. Then the formulation was deaerated and was packed out.

### EXAMPLE 13

### Denture Adhesive

### FORMULATION 1

25.0% CMC 7H3SXF (Hercules Incorporated)
25.0% HMHEC2
45.0% Petrolatum (Snow White from Penerico)
5.0% Mineral oil (Drakeol 9 from Penerico)

### FORMULATION 2

50.0% HMHEC2
45.0% Petrolatum (Snow White from Penerico)
5.0% Mineral oil (Drakeol 9 from Penrico)

Petrolatum was preheated to 60°C in a vessel and mineral oil was added and mixed for five minutes. Polymer was then added to agitated liquid in the vessel and continued to mix for 30 minutes. The formulation was then transferred to a packout container and allowed to cool to about 25°C.

### EXAMPLE 14

### CLEAR STICK ANTIPERSPIRANT

A two phase method was used to prepare the clear stick antiperspirant as follows:

### Phase I

About 65% of the total propylene glycol used (excluding that which is part of the antiperspirant salt solution) was charged to a reaction vessel. HMHEC1 was added to the vessel and stirred well until dissolved. The vessel was heated to dissolve the polymer. Once the polymer was dissolved, the solution was heated to 110°C-115°C, and the dibenzylidine sorbitol was added and mixed until completely dissolved. This Phase I solution was then cooled to about 100°C.

### Phase II

About 35% of the total propylene glycol used (excluding that which is part of the antiperspirant salt solution) was added to the another vessel, stirred and heated to about 60-70°C. The Na₄EDTA was added and mixed well to form a slurry. The antiperspirant salt solution was added next to this vessel and the solution was mixed well until it becomes clear and homogeneous. The emollients, dimethicone copolymer, was added and the Phase II solution was mixed until it became clear.

### Combined Phase:

Phase II was added to Phase I while mixing and cooled to 80°C. Optionally a fragrance would be added at this point and allowed to mix well. The product was poured into a 1 oz. glass jars and allowed to cool overnight. After cooling overnight, the samples were tested for physical and chemical properties.

### Equipment used:

Two 400 ml glass beakers, oil bath, clamps, mechanical stirrer, Jiffy stirrer and thermometer, and a covering to prevent contamination, such as plastic wrap.

| **Total Formulation for this Example** | | |
|---|---|---|
| 1. | Propylene glycol | 49.70 g |
| 2. | Al/Zr tetrachlorohydrate-gly | 36.60 g* |
| 3. | Dibenzylidene sorbitol | 0.50 g |
| 4. | HMHEC1 | 0.30 |
| 5. | Sodium EDTA | 0.20 |
| 6. | Dimethicone copolymer (ABIL B 8851) | 0.25 |
| 7. | Fragrance (optional) | 1.25 |

| | | |
|---|---|---|
| * 30% active solution. | | |

| **Phase I:** | |
|---|---|
| Polypropylene glycol | 32.30 g |
| Dibenzylidene sorbitol | 0.50 g |
| HMHEC1 | 0.30 g |

| **Phase II:** | |
|---|---|
| Polypropylene glycol | 17.40g |
| Al/Zr tetrachlorohydrate-gly | 36.60 g |
| Sodium EDTA | 0.20 g |
| Dimethicone copolymer | 0.25 g |
| Fragrance (optional) | 1.25 g |

### Chemicals, Suppliers:

1. Propylene glycol (EM Science UPS grade)
2. Al/Zr tetrachlorohydrate-gly (Westwood Chemical Co.) Westchlor A2Z 8160 30% PG solution.
3. Dibenzylidene sorbitol (Milliken Chemicals) Millithix 925.
4. HMHEC1 (Hercules Incorporated)
5. Sodium EDTA (Aldrich #5403EJ)
7. Dimethicone copolymer (Goldschmidt Chemical) ABIL B 8851
8. Fragrance

### EXAMPLE 15

### Lubricating Gel

| **Ingredient** | **Weight %** | **Weight (g)** |
|---|---|---|
| HMHEC2 | 1.00 | 4.00 |
| *Klucel® HF | 1.00 | 4.00 |
| Propylene glycol | 97.9 | 391.60 |
| Methylparaben | 0.10 | 0.40 |
| | $\overline{\text{100.00}}$ | $\overline{\text{400.00}}$ |

| | | |
|---|---|---|
| *Klucel HF: Hydroxypropylcellulose from Hercules Incorporated, Wilmington, DE. | | |

### Procedure:

1. The dry blend of HMHEC2 and Klucel HF was dispersed in vigorously agitated propylene glycol and mixed for 10 minutes.
2. Next, the temperature was raised to 90°C and mixed for one hour.
3. Cooled to room temperature while mixing.
4. The preservative methylparaben was added and mixed for 10 minutes.
5. Dispensed into containers.

### COMPARATIVE EXAMPLE I

### Lubricating Gel

The same experiment as in Example 15 was repeated except that **Benecel® MP 943W was used in place of HMHEC2.
**Benecel MP943W: Hydroxypropylmethylcellulose from Hercules Incorporated.

### EXAMPLE 16

### Lubricating Gel

| **Ingredient** | **Weight %** | **Weight (g)** |
|---|---|---|
| HMHEC2 | 1.50 | 7.50 |
| Glycerine, USP | 20.00 | 100.00 |
| Distilled Water | 78.00 | 390.00 |
| Diazolidinyl Urea, PG, Methylparaben, Propylparaben (Germaben II, ISP) | 0.50 | 2.50 |
| | $\overline{\text{100.00}}$ | $\overline{\text{500.00}}$ |

### Procedure:

1. The glycerin while being stirred was heated to 60°C in a water bath and was covered with Saran wrap while heating.
2. The HMHEC2 was dispersed in the heated glycerin by sifting it into the vortex of the stirred glycerin.
3. The distilled water was heated to 60°C in a separate vessel and slowly added to the dispersion and mixed for an additional 30 minutes while maintaining the temperature at 60°C.
4. Next, the preservative was added and cooled to room temperature and the lubricating gel was put in to containers.

### COMPARATIVE EXAMPLE J

### Lubricating Gel

The same experiment as in Example 16 was repeated except that *Natrosol® 250H NF was used in place of HMHEC2.
*Natrosol 250 NF is Hydroxyethylcellulose from Hercules Incorporated.

### EXAMPLE 17

### Denture Adhesive

One hundred-gram batches of denture adhesive were prepared according to the following formulas:

| | |
|---|---|
| Petrolatum | 50.0 |
| HMHEC2 | 50.0 |
| | 100.0 |

### Procedure:

The petrolatum was weighed into a 250 ml beaker. The beaker was placed in a circulating oil bath heated to 67°C. The contents were stirred at a low speed on an electric mixer having two 1-½ inch diameter propellers spaced ¼ inch apart on the shaft. When contents were 65°C, the polymer was added slowly while adjusting the mixer speed to maintain a vortex in the mixture. Mixing was continued for one hour.

### Preparation of Artificial Saliva solution for mechanical testing:

Artificial saliva was prepared according to the following formula:

| **Concentrate:** | |
|---|---|
| Potassium thiocyanate | 2.0 |
| Potassium chloride | 14.0 |
| Sodium phosphate, dibasic, 7-hydrate | 2.0 |
| Sodium phosphate, monobasic, monohydrate | 1.8 |
| Deionized water, boiled | 1000.0 |
| | $\overline{\text{1019.8}}$ |

| **Dilution:** | |
|---|---|
| Concentrate | 1 part by weight |
| Deionized water, boiled | 9 parts by weight |
| | $\overline{\text{10 parts by weight}}$ |

The pH of the dilution was adjusted to 7.0 with sodium phosphate, dibasic, 7-hydrate.

### Testing of Denture Adhesives:

### MTS CYCLIC COMPRESSION/ TENSION TEST:

The apparatus consisted of a specially designed fixture having a 3" diameter Plexiglas upper plate, and a 2" diameter Plexiglas lower plate surrounded by a 4.5" diameter Plexiglas cylinder to form a reservoir. Special adapters were fabricated for mounting in the MTS instrument. Two ml of the DA sample were dispensed from a 5ml disposable syringe and spread evenly onto the lower stage of the test fixture. The fixture reservoir was filled with artificial saliva so as to just barely immerse the surface of the sample (∼180ml). The upper stage of the fixture was brought into contact with the sample, leaving a starting position span of 0.04"above the lower plate, and the instrument was cycled to travel up to a span of 0.06"and down to a span of 0.03"at a crosshead speed of 0.20 in/min. and full scale load of 20 to 50 lbs. The test was operated for 200 cycles, and the tension and compression forces were recorded in Table 4. Formulations were run in triplicate and the averages were reported.¹^{,2}
¹ GAF Chemicals Co., Method and Procedure Number MP-675-W, September 7, 1989.
²Gilbert Banker and David P. DeMagistris, Evaluation of Adhesional Properties of Polymer samples using the Instron Universal Stress/Strain Analyzer, unpublished Purdue University report for GAF, July 10, 1979.

### COMPARATIVE EXAMPLE K

### Denture Adhesive

The same experiment as in Example 17 was repeated that *Natrosol® 250 HX was used in place of HMHEC2.
*Natrosol 250HX is Hydroxyethylcellulose from Hercules Incorporated.

### EXAMPLE 18

### Clear Stick Antiperspirant

A two phase method was used to prepare the clear stick antiperspirant as follows:

### Phase I

About 65% of the total propylene glycol used (excluding that which is part of the antiperspirant salt solution) was charged to a reaction vessel. HMHEC1 was added to the vessel and stirred well until dissolved. The vessel was heated to dissolve the polymer. Once the polymer was dissolved, the solution was heated to 110°C-115°C, and the dibenzylidine sorbitol was added and mixed until completely dissolved. This Phase I solution was then cooled to about 100°C.

### Phase II

About 35% of the total propylene glycol used (excluding that which is part of the antiperspirant salt solution) was added to the another vessel, stirred and heated to about 60-70°C. The Na₄EDTA was added and mixed well to form a slurry. The antiperspirant salt solution was added next to this vessel and the solution was mixed well until it becomes clear and homogeneous. The emollient, dimethicone copolymer, was added and the Phase II solution was mixed until it became clear.

### Combined Phase:

Phase II was added to Phase I while mixing and cooled to 80°C. A fragrance was added at this point and allowed to mix well. The product was poured into a 1 oz. glass jars and allowed to cool ovemight. After cooling overnight, the samples were tested for physical and chemical properties.

### Equipment Used:

Two 400 ml glass beakers, oil bath, clamps, mechanical stirrer, Jiffy stirrer and thermometer, and a covering to prevent contamination, such as plastic wrap.

| **Total Formulation for this Example:** | | |
|---|---|---|
| 1. | Propylene glycol | 49.67g |
| 2. | Al/Zr tetrachlorohydrate-gly | 36.60g* |
| 3. | Dibenzylidene sorbitol | 0.50 g |
| 4. | HMHEC1 | 0.30g |
| 5. | Sodium EDTA | 0.20g |
| 6. | Dimethicone copolymer (ABIL B 8851) | 0.25g |
| | | $\overline{\text{87.45g}}$ |

| | | |
|---|---|---|
| * 30% active solution. | | |

| **Phase I:** | |
|---|---|
| Polypropylene glycol | 32.07 g |
| Dibenzylidene sorbitol | 0.50 g |
| HMHEC1 | 0.30 g |
| | $\overline{\text{32.87g}}$ |

| **Phase II:** | |
|---|---|
| Polypropylene glycol | 17.60g |
| Al/Zr tetrachlorohydrate-gly | 36.60 g |
| Sodium EDTA | 0.20 g |
| Dimethicone copolymer | 0.25 g |
| | $\overline{\text{54.65g}}$ |

### COMPARATIVE EXAMPLE L

### Clear Stick Antiperspirant

The same experiment as in Example 18 was repeated except that CMHEC 420H was used in place of HMHEC1.

| **Raw Materials and Their Sources for Antiperspirant Stick** | |
|---|---|
| **Raw Material** | **Supplier** |
| Propylene glycol (USP Grade) | EM Science Gibbstown, NJ |
| | |
| Al/Zr tetrachlorohydrate-gly Westchlor A2Z 8160 30% PG solution | Westwood Chemical Corporation Middletown, New York |
| | |
| Dibenzylidene sorbitol Millithix 925. | Milliken Chemicals |
| | |
| HMHEC1 | Hercules Incorporated Wilmington, DE |
| | |
| Carboxymethylhydroxyethylcellulose CMHEC420H | Hercules Incorporated |
| | |
| Sodium EDTA Aldrich #5403EJ | Aldrich Chemical Company Milwaukee, Wisconsin |
| | |
| Dimethicone copolymer Corporation ABIL B 8851 | Goldschmidt Chemical Hopewell, VA |

### EXAMPLE 19

### Shampoo

| **Ingredient** | **Weight %** |
|---|---|
| Distilled water | 58.80 |
| Ammonium lauryl sulfate, 30% (Stepanol AM) | 27.50 |
| Disodium cocoamphodiacetate, 50% (Miranol C2M) | 6.90 |
| Sodium laureth sulfate, 60% (Steol CS460) | 5.70 |
| HMHEC2 | 0.60 |
| Germaben II | 0.50 |
| Citric Acid | to pH 5.5 |
| | $\overline{\text{100.00}}$ |

### Procedure:

1. In a water bath, water was heated to 70°C while agitating and was kept covered to prevent moisture loss.
2. The HMHEC2 was sifted slowly into the vortex of the water.
3. The mixture was cooled to 40°C.
3. The remaining ingredients were added, one at a time, while mixing well between each addition.
4. The pH was adjusted to 5.5 with citric acid.

### COMPARATIVE EXAMPLE M

### Shampoo

The same experiment as in Example 19 was repeated except that Benecel MP 943W was used in place of HMHEC2.

| **Materials And Suppliers for Shampoo** | | |
|---|---|---|
| **CTFA adopted Name** | **Trademark** | **Supplier** |
| Ammonium lauryl sulfate | Stepanol AM | Stepan Chemical Co. Northfield, IL |
| | | |
| Disodium cocoamphodiacetate | Miranol C2M | Rhone-Polenc, Cranbury, NJ |
| | | |
| Sodium laureth sulfate | Steol CS460 | Stepan Chemical Co. |
| | | |
| Preservative | Germaben II | Sutton Labs Chatham, NJ |
| | | |
| Hydroxypropylmethylcellulose | Benecel MP943W | Hercules Incorporated Wilmington, DE |

### EXAMPLE 20

### Pearlescent Shampoo

| **Ingredient** | **Weight %** | **Weight (g)** |
|---|---|---|
| Distilled water | q.s. to 100.00 | 347.25 |
| TEA-lauryl sulfate (40% active) (Stepanol WAT) | 15.00 | 75.00 |
| Sodium lauroamphoacetate (and) sodium trideceth sulfate (Miranol MHT) | 10.00 | 50.00 |
| Cocamide DEA (Ninol 40C0) | 2.50 | 12.50 |
| Glycol stearate (Emerest 2400) | 1.20 | 6.00 |
| Propylene glycol (and) diazolidinyl urea (and) methylparaben (and) propylparaben (Germaben II) | 0.75 | 3.75 |
| HMHEC1 | 0.60 | 3.00 |
| N-Hance® 3000 cationic guar | 0.50 | 2.50 |
| Citric acid (50% solution) | pH adjust | ------ |
| | $\overline{\text{100.00}}$ | $\overline{\text{500.00}}$ |

### Procedure:

1. The N-Hance® product was dispersed by adding slowly to the vortex of well-agitated water in a container. The pH was reduced to 7.0 with citric acid solution to promote dissolution of the surface-treated N-Hance and the solution was heated to 50°C.
2. HMHEC1 was slowly sifted into the heated N-Hance® solution and mixed until fully dissolved.
3. The temperature was raised to 70°C. Next, the TEALS and glycol stearate were added, one at a time. Between each addition, the mixture was well agitated. Heat was turned off once it looked homogeneous. The mixing was continued.
4. When the temperature reached 55°C, the remaining ingredients were added, one at a time.
5. Adjusted to pH 5.0 with citric acid solution.
6. Cooled to 40°C and added fragrance.

### COMPARATIVE EXAMPLE N

### Pearlescent Shampoo

Same experiment as in Example 20 was repeated except that Benecel MP943W from Hercules Incorporated was used in place of HMHEC1.

| **List of Ingredients and Their Suppliers for Pearlescent Shampoo** | | |
|---|---|---|
| **CTFA Adopted Name** | **Trademark** | **Supplier** |
| TEA-lauryl sulfate | Stepanol WAT | Stepan Company Nothfield, Illinois |
| | | |
| Sodium lauroamphoacetate (and) sodium trideceth sulfate | Miranol MHT | Rhone-Poulenc Cranbury, NJ |
| | | |
| Cocamide DEA | Ninol 40C0 | Stepan Company Nothfield, IL |
| | | |
| Glycol stearate | Emerest 2400 | Henkel Corporation Hoboken, NJ |
| | | |
| Propylene glycol (and) dizolidinyl urea (and) methylparaben (and) propylparaben | Germaben II | Sutton Lab Chatham, NJ |
| | | |
| Guar Hydroxypropyltrimonium chloride | N-Hance® 3000 | Hercules Incorporated Wilmington, DE |

### EXAMPLE 21

### Sunscreen Lotion

| | **Ingredient** | **Weight %** | **Weight (g)** |
|---|---|---|---|
| A. | Mineral Oil (Klearol, Witco) | 13.00 | 65.00 |
| | Polyoxypropylene 15 Stearyl Ether (Arlamol E, ICI) | 6.00 | 30.00 |
| | Octyl Methoxycinnimate (Neo Heliopan AV, H&R) | 5.00 | 25.00 |
| | Benzophenon-3 (Uvinul M40, BASF) | 3.00 | 15.00 |
| | Hydrogenated Castor Oil (Castor Wax, Ross) | 1.40 | 7.00 |
| | Sorbiton Monoisostearate (Arlacel 987, ICI) | 1.20 | 6.00 |
| | Polyoxyethylene Polyol Fatty Acid Ester (Arlatone T, ICI) | 1.00 | 5.00 |
| | Ozokerite Wax (O Wax 77W, Ross) | 1.00 | 5.00 |
| | Polyoxyethylene Fatty Acid Ester (Arlacel 989, ICI) | 0.50 | 2.50 |
| B. | HMHEC1 | 0.50 | 2.50 |
| | Distilled Water | 63.60 | 318.00 |
| | Glycerine | 3.00 | 15.00 |
| | Magnesium Sulfate | 0.70 | 3.50 |
| | Diazolidinyl Urea, PG, Methylparaben, Propylparaben (Germaben II, ISP) | 0.10 | 0.50 |
| | | $\overline{\text{100.00}}$ | $\overline{\text{500.00}}$ |

### Procedure:

1. All the ingredients of Part A were mixed together in a vessel while stirring.
2. The temperature was raised to 70°C, and the mixture was stirred for 30 minutes.
3. For Part B, HMHEC1 was dispersed in the distilled water. The slurry pH was raised to 8.5 with NaOH and mixed until dissolved. Glycerin, magnesium sulfate, and the preservative were added one at a time while mixing. The mixture was stirred between each addition to make sure there were no lumps.
4. Part B was added to Part A slowly while stirring and stirred 30 minutes at 70°C.
5. This new mixture was cooled to room temperature while stirring and filled into containers.

### COMPARATIVE EXAMPLE O

### Sunscreen Lotion

The same experiment as in Example 21 was repeated except that CMC 7HF NF was used in place of HMHEC1.

| **Materials and Their Suppliers for Sunscreen Lotion** | | |
|---|---|---|
| **CTFA Adopted Name** | **Trademark** | **Supplier** |
| Mineral Oil | Klearol | Witco Corporation Dublin, OH |
| | | |
| Polyoxypropylene 15 Stearyl Ether | Arlamol E | ICI Surfactants Wilmington, DE |
| | | |
| Octyl Methoxycinnimate | Neo Heliopan AV | H&R Corporation Springfield, NJ |
| | | |
| Benzophenon-3 | Uvinul M40 | BASF Corporation Washington, NJ |
| | | |
| Hydrogenated Castor Oil | Castor Wax | Ross |
| | | |
| Sorbiton Monoisostearate | Arlacel 987 | ICI Surfactants |
| | | |
| Polyoxyethylene Polyol Fatty Acid Ester | Arlatone T | ICI Surfactants |
| | | |
| Ozokerite Wax | O Wax 77W | Ross |
| | | |
| Polyoxyethylene Fatty Acid Ester | Arlacel 989 | ICI Surfactants |
| | | |
| Diazolidinyl Urea, PG, Methylparaben, Propylparaben | Germaben II | Sutton Labs Chatham, NJ |
| | | |
| Carboxymethyl cellulose | CMC 7HF NF | Hercules Incorporated |

### EXAMPLE 22

### Hydro-Alcoholic Roll-On

| **Part** | **Ingredients** | **Weight %** | **Weight (g)** |
|---|---|---|---|
| A | REACH® 501 Solution (50% Al chlorohydrate) | 40.00 | 160.00 |
| B | Procetyl AWS (PPG-5 ceteth-20) | 2.00 | 8.00 |
| C | HMHEC4 | 0.20 | 0.80 |
| D | Deionized water | 15.70 | 62.80 |
| E | SD Alcohol 40 | 41.10 | 164.40 |
| F | Fragrance (d) | 1.00 | 4.00 |
| | | $\overline{\text{100.00}}$ | $\overline{\text{400.00}}$ |

### Procedure:

1. HMHEC4 was dispersed into the deionized water (D) in a container and the pH of the dispersion was adjusted to 8.5 with NaOH solution. The dispersion was then mixed for 30 minutes.
2. Gradually component A was added to the dispersion and mixed rapidly using overhead stirring to dissolve.
3. In a separate container components B and E were combined and then added slowly with constant agitation to the rest of the batch.
4. Then the fragrance was aded and mixed for 5 minutes.
5. The resulting mixture was poured into roll-on containers.

### COMPARATIVE EXAMPLE P

### Hydro-Alcohollc Roll-On

The same experiment was run as in Example 22 except that Natrosol 250MR CS from Hercules Incorporated was used in place of HMHEC4.

| **Materials and Their Suppliers for Hydro-alcoholic Roll-on** | | |
|---|---|---|
| **CTFA Adopted Name** | **Trademark** | **Supplier** |
| Aluminum chlorohydrate | REACH 501 | Rehies Incorporation Berkeley Height, NJ |
| | | |
| PPG-5 ceteth-20 | Procetyl AWS | Croda Incorporation Parsippany, NJ |
| | | |
| Ethyl alcohol | SD Alcohol 40 | |
| Fragrance Classic oriental/spice | #Q-7148 | Quest International Fragrances Inc. Mount Olive, NJ |
| | | |
| Hydroxyethylcellulose | Natrosol 250MR CS | Hercules Incorporated Wilmington, DE |

### EXAMPLE 23

### Shower Gel

| **Ingredient** | | **Weight %** | **Weight (g)** |
|---|---|---|---|
| A. | Deionized water | q.s. to 100.00 | 282.10 |
| B. | HMHEC1 (Hercules Inc.) | 0.95 | 4.75 |
| C. | Sodium Laureth Sulfate (Steol CS460, Stepan) | 11.53 | 57.65 |
| | Disodium Laureth Sulfosuccinate (Stepan Mild SL3, Stepan) | 11.80 | 59.00 |
| | Disodium Cocoamphodiacetate (Miranol C2M Conc NP, Rhone-Poulenc) | 6.00 | 30.00 |
| | Sodium Lauroyl Sarcosinate (Crodasinic LS 30, Croda) | 7.25 | 36.25 |
| | Propylene Glycol | 2.00 | 10.00 |
| | Quaternized Wheat Protein (WheataFlor, Croda) | 1.00 | 5.00 |
| | Hydrolysate and Hydrolyzed Wheat Protein and Wheat Germ Oil & Polysorbate 20 Glycol Distearate and Laureth-4 and CAPB (Euperlan PK 3000, Henkel) | 2.00 | 10.00 |
| | Disodium EDTA (EDETA BD, BASF) | 0.10 | 0.50 |
| | Perfume (Drom 229033, Drom) | 0.35 | 1.75 |
| | Phenoxyethanol and Methylparaben and Ethylparaben and Propylparaben and Butylparaben (Phinonip, Nipa) | 0.60 | 3.00 |
| | | $\overline{\text{100.00}}$ | $\overline{\text{500.00}}$ |

### Procedure:

1. HMHEC was dispersed into well agitated water to form a slurry.
2. The pH of the slurry was adjusted to 8.5 with a NaOH solution and the slurry was mixed until a solution was formed and had no lumps. Next, the ingredients of Phase C were added to the solution in the order listed above while mixing for one minute between each addition or until the mixture became homogeneous.
3. The pH of the final gel product was adjusted to 5.3-5.7 and filled into containers.

### COMPARATIVE EXAMPLE Q

### Shower Gel

The same experiment as in Example 23 was run except that N-Hance 3196 was used in place of HMHEC1; the polymer was dispersed in water and mixed for five minutes. The slurry pH was lowered to below 6.0 to 7.0 and mixed for one hour or until no lumps were observed, followed by the addition of Phase C as above.

| **Raw Materials and Their Sources For Shower Gel** | | |
|---|---|---|
| **CTFA Adopted Name** | **Trade Name** | **Supplier** |
| Guar Hydroxypropyltrimonium Chloride | N-Hance® 3196 | Hercules Incorporated Wilmington, DE |
| | | |
| Sodium Laureth Sulfate | Steol CS460 | Stepan Company Nothfield, NJ |
| | | |
| Disodium Laureth Sulfosuccinate | Stepan Mild SL3 | Stepan Company |
| | | |
| Disodium Cocoamphodiacetate | Miranol C2M Conc NP | Rhone-Poulenc Cranbury, NJ |
| | | |
| Sodium Lauroyl Sarcosinate | Crodasinic LS 30 | Croda Incorporated Parsipanny, NJ |
| | | |
| Quaternized Wheat Protein | WheataFlor | Croda Incorporated |
| | | |
| Hydrolysate and Hydrolyzed Wheat Protein and Wheat Germ Oil & Polysorbate 20 Glycol Distearate and Laureth-4 & CAPB | Euperlan PK 3000 | Henkel Corporation Hoboken, NJ |
| | | |
| Disodium EDTA | EDETA BD | BASF Corporation Washington, NJ |
| | | |
| Perfume | Drom 229033 | Drom International Towaco, NJ |
| | | |
| Phenoxyethanol and Methylparaben & Ethylparaben & Propylparaben and Butylparaben | Phinonip | Nipa Hardwicke Inc. Wilmington, DE |
| | | |
| Guar Hydroxypropyl trimoniumchloride | N-Hance 3196 | Hercules Incorporated Wilmington, DE |

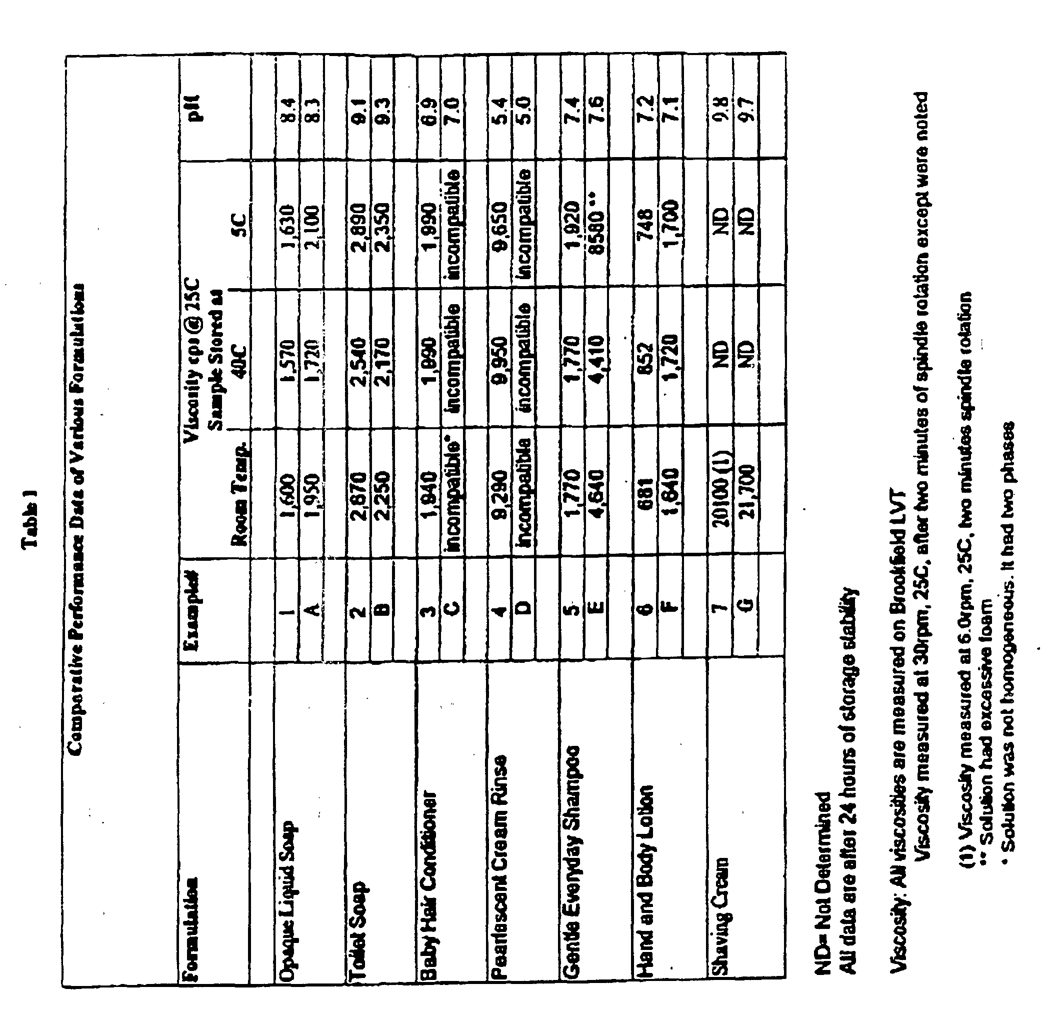

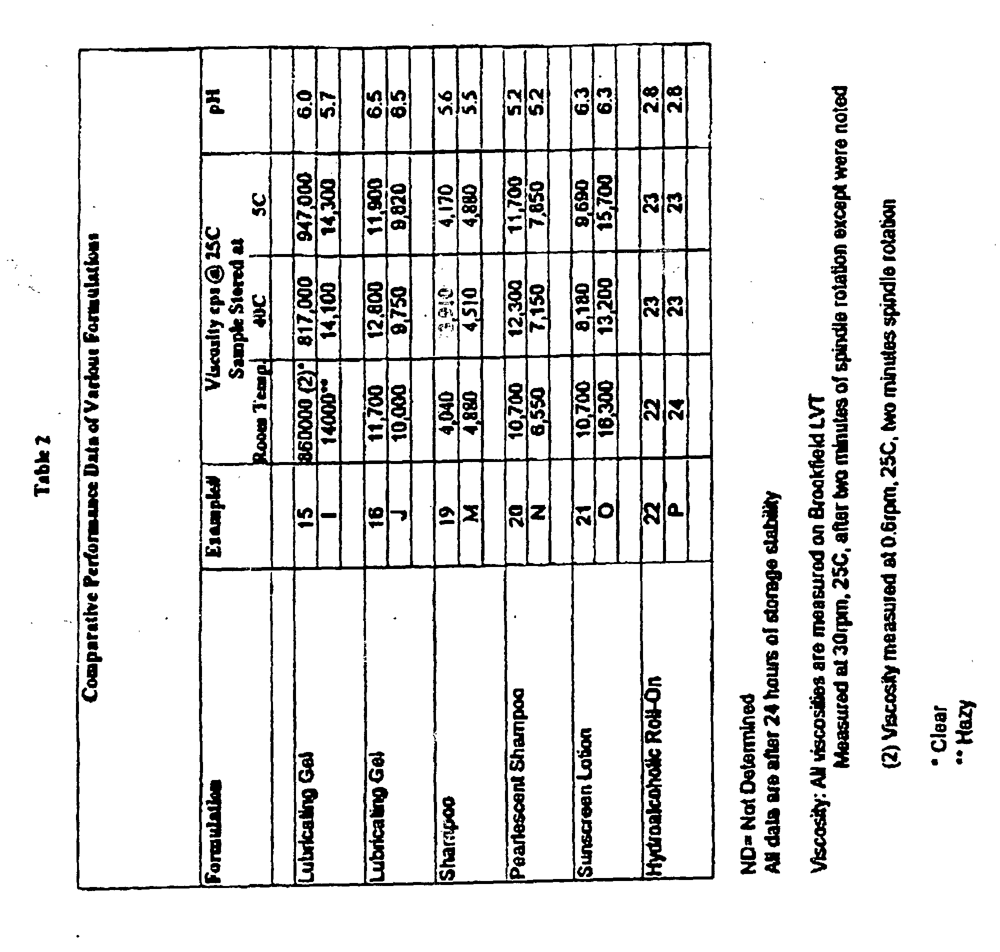

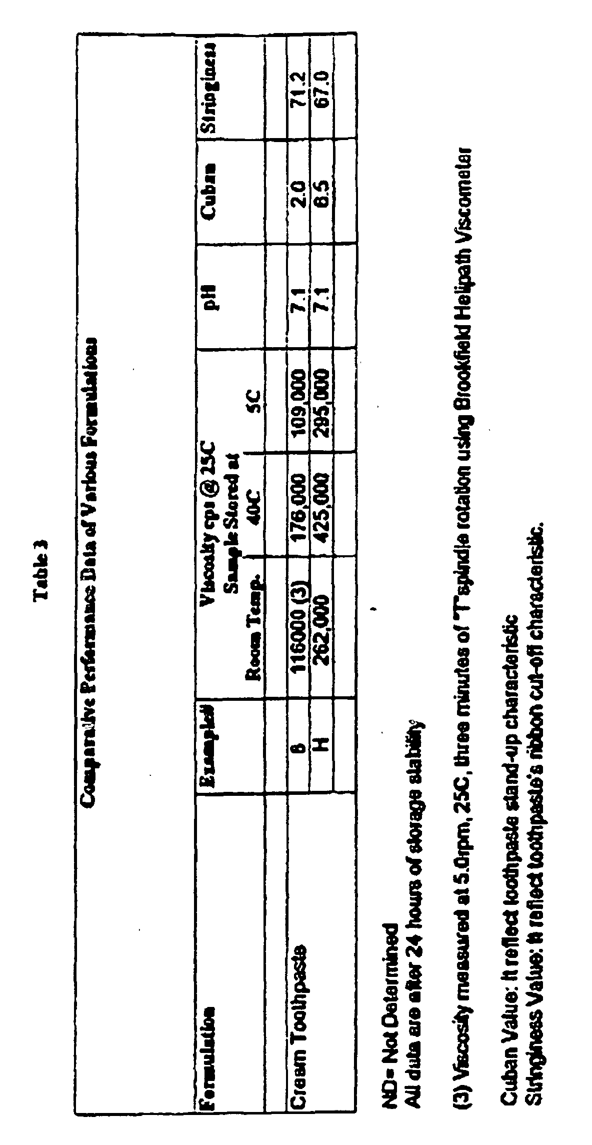

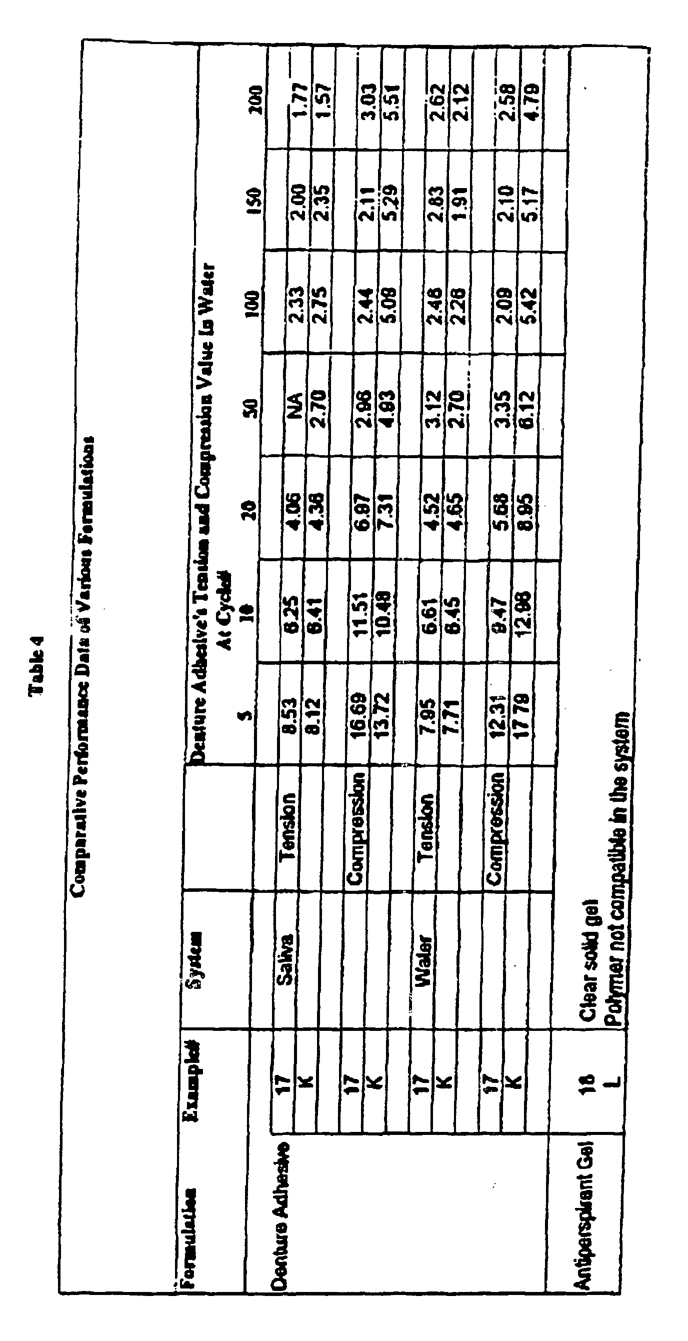

## Claims

1. A personal care composition comprising:
(a) from 0.1 % to 99 % by weight of a vehicle system which comprises a hydrophobically modified nonionic water soluble polysaccharide polymer which comprises a water soluble polysaccharide polymer backbone and a hydrophobic moiety selected from the group consisting of a 3-a!koxy-2-hydroxypropyl group wherein the alkyl moiety is a straight or branch chain having 2-6 carbon atoms, C₃-C₇ alkyl, aryl alkyl, alkyl aryl groups and mixtures thereof, wherein the ratio of the hydrophilic portion to the hydrophobic portion of the polymer is from 2:1 to 1000:1, and
(b) at least one other personal care ingredient.

2. The composition of claim 1 wherein the composition also comprises from 0.01 % to 25% by weight of the personal care composition of a surfactant.

3. The composition of claim 2 wherein the surfactant is selected from the group consisting of anionic, nonionic, cationic, zwitterionic, amphoteric, and mixtures thereof.

4. The composition of claim 1 wherein the composition also comprises from 0.1 % to 99 % by weight of the personal care composition of a compatible solvent or solvent mixture.

5. The composition of claim 4 wherein the solvent or solvent mixture is selected from the group consisting of water, water-lower alkanols mixtures, polyhydric alcohols having from 3 to 6 carbon atoms and from 2 to 6 hydroxyl groups, and mixtures thereof.

6. The composition of claim 5 wherein the solvent or solvent mixture is selected from the group consisting of water, propylene glycol, water-glycerine, sorbitol-water, water-ethanol, and mixtures thereof.

7. The composition of claim 2 wherein the composition also comprises from 0.1% to 99% by weight of the personal care composition of a compatible solvent or solvent mixture.

8. The composition of claim 1 wherein the hydrophobically modified polysaccharide backbone is selected from the group of hydroxyethylcellulose (HEC), hydroxypropylcellulose (HPC), methylcellulose (MC), hydroxypropylmethylcellulose (HPMC), ethylhydroxyethylcellulose (EHEC), and methylhydroxyethylcellulose (MHEC), and agar, dextran, locust bean gum, starch, guar, and their nonionic derivatives, and mixtures thereof.

9. The composition of claim 1 wherein the polysaccharide backbone is HEC and the hydrophobic moiety is 3-butoxy-2-hydroxypropyl.

10. The composition of claim 4 wherein the composition also contains an effective viscosifying amount of a salt.

11. A hair or skin care composition comprising a solvent and an effective amount of the personal care composition of claim 7.

12. A shampoo comprising an effective amount of the personal care composition of claim 4.

13. A conditioner comprising an effective amount of the personal care composition of claim 4.

14. A shampoo-conditioner comprising an effective amount of the composition of claim 1.

15. A sun care product comprising a solvent and an effective amount of the personal care composition of claim 1.

16. A shower gel comprising an effective amount of the cleaning composition of claim 1.

17. A soap comprising an effective amount of the personal care composition of claim 1.

18. A hair styling gel composition comprising an effective amount of the personal care composition of claim 1.

19. A hair styling gel composition comprising an effective amount of the personal care composition of claim 4.

20. A hair anti-dandruff composition comprising a solvent and an effective amount of the personal care composition of claim 7.

21. A hair growth promoter composition comprising an effective amount of the personal care composition of claim 1.

22. A hair colorant composition comprising an effective amount of the personal care composition of claim 1.

23. A hair bleaching agent composition comprising an effective amount of the personal care composition of claim 1.

24. A hair antifrizzing agent composition comprising an effective amount of the personal care composition of claim 1.

25. A hair relaxer composition comprising an effective amount of the personal care composition of claim 1.

26. A dentifrice composition comprising an effective amount of the personal care composition of claim 1.

27. A mouth wash compositlon comprising an effective amount of the personal care composition of claim 1.

28. A denture adhesive composition comprising an effective amount of the personal care composition of claim 1.

29. A shaving product composition comprising an effective amount of the personal care composition of claim 1.

30. A lubricating gel composition comprising an effective amount of the personal care composition of claim 1.

31. A spermicide gel composition comprising an effective amount of the personal care composition of claim 1.

32. A beauty aid composition comprising an effective amount of the personal care composition of claim 1.

33. An underarm solid stick composition comprising an effective amount of the personal care composition of claim 1.

34. An underarm gel composition comprising an effective amount of the personal care composition of claim 1.

35. An underarm liquid composition comprising an effective amount of the personal care composition of claim 1.

36. An underarm liquid composition of claim 35 wherein the composition comprises an aerosol ingredient.

37. The composition of claim 1 wherein the composition also comprises an oil-in-water or water in oil emulsion.

38. A cleansing composition comprising an effective amount of the composition of claim 1.

39. A hair grooming and hair detangler composition comprising an effective amount of the personal care composition of claim 1.

40. A razor blade lubrication strip compostion comprising an effective amount of the personal care composition of claim 1.

41. A cleansing tissue composition comprising an effective amount of the personal care composition of claim 1.

## Patentansprüche

1. Körperpflegezusammensetzung, umfassend:
(a) 0,1 bis 99 Gew.% eines Trägersystems, das ein hydrophob modifiziertes, nicht-ionisches, wasserlösliches Polysaccharidpolymer umfasst, das ein wasserlösliches Polysaccharidpolymergerüst und eine hydrophobe Einheit umfasst, die aus der Gruppe ausgewählt ist, die aus einer 3-Alkoxy-2-hydroxypropyl-Gruppe, worin die Alkyleinheit eine lineare oder verzweigte Kette mit 2 bis 6 Kohlenstoffatomen ist, C₃₋₇-Alkyl-, Arylalkyl-, Alkylarylgruppen und Mischungen daraus besteht, worin das Verhältnis des hydrophilen Anteils zum hydrophoben Anteil des Polymers 2:1 bis 1.000:1 beträgt, und
(b) wenigstens einen anderen Körperpflegebestandteil.

2. Zusammensetzung gemäss Anspruch 1, worin die Zusammensetzung ebenfalls 0,01 bis 25 Gew.% der Körperpflegezusammensetzung an einem Tensid umfasst.

3. Zusammensetzung gemäss Anspruch 2, worin das Tensid aus der Gruppe ausgewählt ist, die aus anionischen, nicht-ionischen, kationischen, zwitterionischen, amphoteren und Mischung daraus besteht.

4. Zusammensetzung gemäss Anspruch 1, worin die Zusammensetzung ebenfalls 0,1 bis 99 Gew.% der Körperpflegezusammensetzung an einem kompatiblen Lösungsmittel oder Lösungsmittelgemisch umfasst.

5. Zusammensetzung gemäss Anspruch 4, worin das Lösungsmittel oder Lösungsmittelgemisch aus der Gruppe ausgewählt ist, die aus Wasser, Mischungen aus Wasser und niederen Alkanolen, mehrwertigen Alkoholen mit 3 bis 6 Kohlenstoffatomen und 2 bis 6 Hydroxylgruppen und Mischungen daraus besteht.

6. Zusammensetzung gemäss Anspruch 5, worin das Lösungsmittel oder Lösungsmittelgemisch aus der Gruppe ausgewählt ist, die aus Wasser, Propylenglykol, Wasser-Glycerin, Sorbit-Wasser, Wasser-Ethanol und Mischungen daraus besteht.

7. Zusammensetzung gemäss Anspruch 2, worin die Zusammensetzung ebenfalls 0,1 bis 99 Gew.% der Körperpflegezusammensetzung an einem kompatiblen Lösungsmittel oder Lösungsmittelgemisch umfasst.

8. Zusammensetzung gemäss Anspruch 1, worin das hydrophob modifizierte Polysaccharidgerüst aus der Gruppe aus Hydroxyethylcellulose (HEC), Hydroxypropylcellulose (HPC), Methylcellulose (MC), Hydroxypropylmethylcellulose (HPMC), Ethylhydroxyethylcellulose (EHEC) und Methylhydroxyethylcellulose (MHEC) und Agar, Dextran, Johannisbrotkernmehl, Stärke, Guar und deren nicht-ionischen Derivaten und Mischungen daraus ausgewählt ist.

9. Zusammensetzung gemäss Anspruch 1, worin das Polysaccharidgerüst HEC ist und die hydrophobe Einheit 3-Butoxy-2-hydroxypropyl ist.

10. Zusammensetzung gemäss Anspruch 4, worin die Zusammensetzung ebenfalls eine effektive, viskositätserzeugende Menge eines Salzes enthält.

11. Haar- oder Hautpflegezusammensetzung, die ein Lösungsmittel und eine effektive Menge der Körperpflegezusammensetzung gemäss Anspruch 7 umfasst.

12. Shampoo, das eine effektive Menge der Körperpflegezusammensetzung gemäss Anspruch 4 umfasst.

13. Haarspülung, die eine effektive Menge der Körperpflegezusammensetzung gemäss Anspruch 4, umfasst.

14. Shampoo-Haarspülung, die eine effektive Menge der Zusammensetzung gemäss Anspruch 1 umfasst.

15. Sonnenpflegeprodukt, das ein Lösungsmittel und eine effektive Menge der Körperpflegezusammensetzung gemäss Anspruch 1 umfasst.

16. Duschgel, das eine effektive Menge der Reinigungszusammensetzung gemäss Anspruch 1 umfasst.

17. Seife, die eine effektive Menge der Körperpflegezusammensetzung gemäss Anspruch 1 umfasst.

18. Haarformgebungsgelzusammensetzung, die eine effektive Menge der Körperpflegezusammensetzung gemäss Anspruch 1 umfasst.

19. Haarformgebungsgelzusammensetzung, die eine effektive Menge der Körperpflegezusammensetzung gemäss Anspruch 4 umfasst.

20. Haar-Antischuppenzusammensetzung, die ein Lösungsmittel und eine effektive Menge der Körperpflegezusammensetzung gemäss Anspruch 7, umfasst.

21. Zusammensetzung zur Förderung des Haarwuchses, die eine effektive Menge der Körperpflegezusammensetzung gemäss Anspruch 1 umfasst.

22. Haarfärbezusammensetzung, die eine effektive Menge der Körperpflegezusammensetzung gemäss Anspruch 1 umfasst.

23. Haarbleichungsmittelzusammensetzung, die eine effektive Menge der Körperpflegezusammensetzung gemäss Anspruch 1 umfasst.

24. Haar-Antikräuselmittelzusammensetzung, die eine effektive Menge der Körperpflegezusammensetzung gemäss Anspruch 1 umfasst.

25. Haar-Lockerungszusammensetzung, die eine effektive Menge der Körperpflegezusammensetzung gemäss Anspruch 1 umfasst.

26. Zahnputzzusammensetzung, die eine effektive Menge der Körperpflegezusammensetzung gemäss Anspruch 1 umfasst.

27. Mundspülungszusammensetzung, die eine effektive Menge der Körperpflegezusammensetzung gemäss Anspruch 1 umfasst.

28. Gebisshaftmittelzusammensetzung, die eine effektive Menge der Körperpflegezusammensetzung gemäss Anspruch 1 umfasst.

29. Rasierproduktzusammensetzung, die eine effektive Menge der Körperpflegezusammensetzung gemäss Anspruch 1 umfasst.

30. Gleitgelzusammensetzung, die eine effektive Menge der Körperpflegezusammensetzung gemäss Anspruch 1 umfasst.

31. Spermizide Gelzusammensetzung, die eine effektive Menge der Körperpflegezusammensetzung gemäss Anspruch 1 umfasst.

32. Schönheitshilfezusammensetzung, die eine effektive Menge der Körperpflegezusammensetzung gemäss Anspruch 1 umfasst.

33. Feste Stiftzusammensetzung für die Achselhöhle, die eine effektive Menge der Körperpflegezusammensetzung gemäss Anspruch 1 umfasst.

34. Gelzusammensetzung für die Achselhöhle, die eine effektive Menge der Körperpflegezusammensetzung gemäss Anspruch 1 umfasst.

35. Flüssige Zusammensetzung für die Achselhöhle, die eine effektive Menge der Körperpflegezusammensetzung gemäss Anspruch 1 umfasst.

36. Flüssige Zusammensetzung für die Achselhöhle gemäss Anspruch 35, worin die Zusammensetzung einen Aerosolbestandteil umfasst.

37. Zusammensetzung gemäss Anspruch 1, worin die Zusammensetzung ebenfalls eine Öl-in-Wasser- oder Wasser-in-Öl-Emulsion umfasst.

38. Reinigungszusammensetzung, die eine effektive Menge der Zusammensetzung gemäss Anspruch 1 umfasst.

39. Haarpflege- und Haarentwirrungszusammensetzung, die eine effektive Menge der Körperpflegezusammensetzung gemäss Anspruch 1 umfasst.

40. Gleitstreifenzusammensetzung für eine Rasierklinge, die eine effektive Menge der Körperpflegezusammensetzung gemäss Anspruch 1 umfasst.

41. Reinigungstuchzusammensetzung, die eine effektive Menge der Körperpflegezusammensetzung gemäss Anspruch 1 umfasst.

## Revendications

1. Composition pour soins corporels, comprenant :
(a) 0,1 % à 99 % en poids d'un système de véhicule qui comprend un polymère polysaccharidique hydrosoluble non ionique à modification hydrophobe qui comprend un squelette polymère polysaccharidique hydrosoluble et un groupement hydrophobe choisi dans le groupe consistant en un groupe 3-alkoxy-2-hydroxypropyle dans lequel le groupement alkyle est une chaîne droite ou ramifiée ayant 2 à 6 atomes de carbone, des groupes alkyle en C₃ à C₇, arylalkyle, alkylaryle et leurs mélanges, le rapport de la partie hydrophile à la partie hydrophobe du polymère étant compris dans l'intervalle de 2:1 à 1000:1, et
(b) au moins un autre ingrédient pour soins corporels.

2. Composition suivant la revendication 1, qui comprend également 0,01 % à 25 %, en poids de la composition pour soins corporels, d'un agent tensioactif.

3. Composition suivant la revendication 2, dans laquelle l'agent tensioactif est choisi dans le groupe consistant en des agents tensioactifs anioniques, non ioniques, cationiques, zwitterioniques, amphotères et leurs mélanges.

4. Composition suivant la revendication 1, qui comprend également 0,1 % à 99 % en poids de la composition pour soins corporels, d'un solvant compatible ou mélange de solvants compatibles.

5. Composition suivant la revendication 4, dans laquelle le solvant ou mélange de solvants est choisi dans le groupe consistant en l'eau, des mélanges eau-alcanol inférieur, des alcools polyhydroxyliques ayant 3 à 6 atomes de carbone et 2 à 6 groupes hydroxyle et leurs mélanges.

6. Composition suivant la revendication 5, dans laquelle le solvant ou mélange de solvants est choisi dans le groupe consistant en l'eau, le propylèneglycol, des mélanges eau-glycérol, sorbitol-eau, eau-éthanol et leurs mélanges.

7. Composition suivant la revendication 2, qui comprend également 0,1 % à 99 %, en poids de la composition pour soins corporels, d'un solvant compatible ou mélange de solvants compatibles.

8. Composition suivant la revendication 1, dans laquelle le squelette polysaccharidique à modification hydrophobe est choisi dans le groupe consistant en hydroxyéthylcellulose (HEC), hydroxypropylcellulose (HPC), méthylcellulose (MC), hydroxypropylméthylcellulose (HPMC), éthylhydroxyéthylcellulose (EHEC) et méthylhydroxyéthylcellulose (MHEC), et gélose, dextrane, gomme de caroube, amidon, gomme guar et leurs dérivés non ioniques, ainsi que leurs mélanges.

9. Composition suivant la revendication 1, dans laquelle le squelette polysaccharidique consiste en HEC et le groupement hydrophobe est un groupement 3-butoxy-2-hydroxypropyle.

10. Composition suivant la revendication 4, qui contient également une quantité, efficace à des fins d'augmentation de viscosité, d'un sel.

11. Composition pour les soins capillaires ou soins cutanés, comprenant un solvant et une quantité efficace de la composition pour soins corporels de la revendication 7.

12. Shampooing comprenant une quantité efficace de la composition pour soins corporels de la revendication 4.

13. Lotion capillaire comprenant une quantité efficace de la composition pour soins corporels de la revendication 4.

14. Shampooing-lotion capillaire comprenant une quantité efficace de la composition de la revendication 1.

15. Produit écran solaire comprenant un solvant et une quantité efficace de la composition pour soins corporels de la revendication 1.

16. Gel pour la douche, comprenant une quantité efficace de la composition nettoyante de la revendication 1.

17. Savon comprenant une quantité efficace de la composition pour soins corporels de la revendication 1.

18. Composition de gel coiffant comprenant une quantité efficace de la composition pour soins corporels de la revendication 1.

19. Composition de gel coiffant comprenant une quantité efficace de la composition pour soins corporels de la revendication 4.

20. Composition capillaire anti-pellicules comprenant un solvant et une quantité efficace de la composition pour soins corporels de la revendication 7.

21. Composition d'activateur de croissance capillaire comprenant une quantité efficace de la composition pour soins corporels de la revendication 1.

22. Composition de colorant capillaire comprenant une quantité efficace de la composition pour soins corporels de la revendication 1.

23. Composition d'agent de décoloration capillaire, comprenant une quantité efficace de la composition pour soins corporels de la revendication 1.

24. Composition d'agent défrisant capillaire comprenant une quantité efficace de la composition pour soins individuels de la revendication 1.

25. Composition de relâchement capillaire comprenant une quantité efficace de la composition pour soins corporels de la revendication 1.

26. Composition de. dentifrice comprenant une quantité efficace de la composition pour soins corporels de la revendication 1.

27. Composition de bains de bouche comprenant une quantité efficace de la composition pour soins corporels de la revendication 1.

28. Composition d'adhésif pour prothèses dentaires, comprenant une quantité efficace de la composition pour soins corporels de la revendication 1.

29. Composition de produit de rasage, comprenant une quantité efficace de la composition pour soins corporels de la revendication 1.

30. Composition de gel lubrifiant comprenant une quantité efficace de la composition pour soins corporels de la revendication 1.

31. Composition de gel spermicide comprenant une quantité efficace de la composition pour soins corporels de la revendication 1.

32. Composition d'adjuvant pour les soins de beauté, comprenant une quantité efficace de la composition pour soins corporels de la revendication 1.

33. Composition en bâtonnet solide pour les aisselles, comprenant une quantité efficace de la composition pour soins corporels de la revendication 1.

34. Composition de gel pour les aisselles, comprenant une quantité efficace de la composition pour soins corporels de la revendication 1.

35. Composition liquide pour les aisselles, comprenant une quantité efficace de la composition pour soins corporels de la revendication 1.

36. Composition liquide pour les aisselles suivant la revendication 35, ladite composition comprenant un ingrédient d'aérosol.

37. Composition suivant la revendication 1, la composition comprenant également une émulsion huile-dans-eau ou eau-dans-huile.

38. Composition nettoyante comprenant une quantité efficace de la composition de la revendication 1.

39. Composition pour soins capillaires et démêlants comprenant une quantité efficace de la composition pour soins corporels de la revendication 1.

40. Composition pour bande lubrifiante de lame de rasoir, comprenant une quantité efficace de la composition pour soins corporels de la revendication 1.

41. Composition pour lingettes démaquillantes, comprenant une quantité efficace de la composition pour soins corporels de la revendication 1.
